# EUROPEAN PATENT SPECIFICATION

(11) **EP 1 599 025 B1**
(45) Date of publication and mention of the grant of the patent: **22.10.2008**
(21) Application number: 05076637.7
(22) Date of filing: 14.05.2002
(51) Int. Cl.: H04M 1/725

(54) **Portable communication device**
Tragbares Kommunikationsgerät
Appareil de communication portable

(30) Priority: 14.05.2001 GB 0111722; 10.12.2001 GB 0129491; 10.12.2001 GB 0129490; 10.04.2002 GB 0208275
(43) Date of publication of application: 23.11.2005
(62) Divisional of application: 02769514.7
(73) Proprietor: Innovision Research & Technology PLC, Cirencester, Gloucestershire GL7 1RQ (GB)
(72) Inventor: White, Andrew, Innovision Research & Tech. PLC, Wokingham, Berkshire RG40 1XS (GB); Borrett, Marc A., Innovision Research & Tech. PLC, Wokingham, Berkshire RG40 1XS (GB); Mapleston, David, Innovision Research & Tech. PLC, Wokingham, Berkshire RG40 1XS (GB)
(74) Representative: Clark, Jane Anne

(56) References cited:
- WO-A-00/79771

## Description

This invention relates to portable communications devices such as telephones arranged to operate using a mobile telecommunications network and to attachable components for such portable communications devices.

Telephones that operate using a mobile telecommunications network are variously known as mobile telephones, cellular telephones and cellphones. For simplicity, the term "mobile telephone" will be used hereinafter.

The provision of mobile telephone fascia that may be changed by the end user for, for example, aesthetic or decorative reasons has become popular in recent years.

WO 00/79771 describes an interchangeable battery pack for a mobile telephone. The battery pack comprises a radio interface, for example, an RFID element, a GPS receiver, a radio times signal receiver etc. Different battery packs, each functioning in accordance with a different communications protocol may be provided.

In one aspect, the present invention provides a portable communications device comprising a first body portion and a second body portion, wherein:
the first body portion has a transceiver for enabling transmission and reception of communications over a mobile telecommunications network, first control means for controlling operation of the device, and signal supplying means for supplying a carrier signal;
the first body portion and the second body portion have respective first and second wireless coupling means arranged in close proximity to one another to enable wireless coupling of the carrier signal from the first wireless coupling means to the second wireless coupling means;
the second body portion carries a data storage device having a memory storing data, power supply deriving means for deriving a power supply from a carrier signal coupled to the second wireless coupling means, second control means for reading data from the memory in response to derivation of a power supply by the power supply deriving means, and modulating means for modulating the carrier signal in accordance with data read from the memory;
the first body portion has data extraction means for extracting data from the modulation of the carrier signal and data supplying means for supplying the data to the first control means to affect a function relating to the operation of the portable communications device.

In an embodiment, the second body portion comprises a fascia of the device and the first body portion is a main body of the device. In an embodiment, the device is a mobile telephone. The mobile telephone fascia may provide the mobile telephone with additional, different or modified functionality or operating characteristics. Different mobile telephone fascias may affect a mobile telephone differently. For example, access to functions or operating characteristics of the mobile telephone may be limited or controlled by the particular fascia fitted to the mobile telephone.

In an embodiment, the data storage device carries control data (which may be software data and/or information data) that is supplied to a main body of the mobile telephone when the fascia is attached to and/or detached from the main body and which affects the functioning and/or operating characteristics of the mobile telephone.

As used herein the term "passive data storage device" means a device that is not self-powered but that derives power from the main body when the fascia is attached to the main body.

In an embodiment, the control data may comprise data for at least one of: graphics; mobile telephone services; subscription services; network services; services; promotions; and advertisement; fascia identification data; games software for games that may be played on the mobile telephone including a new game or modifications for such games software already installed on the mobile telephone; and data for enabling a user to access any one or more of the above types of data using their mobile telephone, for example an access code to enable access to control data already stored by the mobile telephone or data such as a telephone number, Internet address or WAP address from which control data can be accessed, or the user's mobile network provider or another third party service provider.

In an embodiment, the data storage device may be a writable data storage device into which, for example, historical data such as use data may be written. Such data may be accumulated by the main body of the mobile telephone which may control the writing of this data into the data storage device. Data may also be written into a SIM card memory.

In an embodiment, the mobile telephone fascia is provided with apertures through which key pads of a user interface of the telephone project.

In another embodiment, the mobile telephone fascia carries a user interface. The user interface may be at least one of a keyboard, touch screen and so on. Different fascias may carry different types of user interfaces.

The use of a data storage device that does not require its own power supply enables costs to be kept down and, moreover, because such passive data storage devices are relatively cheap to produce and can be incorporated into products relatively easily (for example during a plastics moulding process), the incorporation of the passive data storage device does not add significantly to costs.

Embodiments of the present invention will now be described, by way of example, with reference to the accompanying drawings, in which:
Figure 1 shows a diagrammatic perspective view of a mobile telephone with a removable fascia embodying the invention separated from a main body of the mobile telephone;
Figure 2 shows a functional block diagram of functional components of a mobile telephone embodying the invention, including a reader unit for enabling detection of a replacement fascia;
Figure 3 shows a functional block diagram of an embodiment of the reader unit shown in Figure 2;
Figure 4 shows a functional block diagram of an example of a passive data storage device;
Figure 5 shows a more detailed functional block diagram of an embodiment of the reader unit;
Figure 6 shows a more detailed functional block diagram of one example of a passive data storage device;
Figure 7 shows a functional block diagram of another example of a passive data storage device;
Figure 8 shows a functional block diagram of another example of a mobile telephone embodying the present invention;
Figure 9 shows a functional block diagram of an example of a passive data storage device for use as a detectable control device shown in Figure 8;
Figures 10 to 12 are very diagrammatic views of examples of user input devices; and
Figure 13 shows a block diagram illustrating a portable communication device embodying the invention.

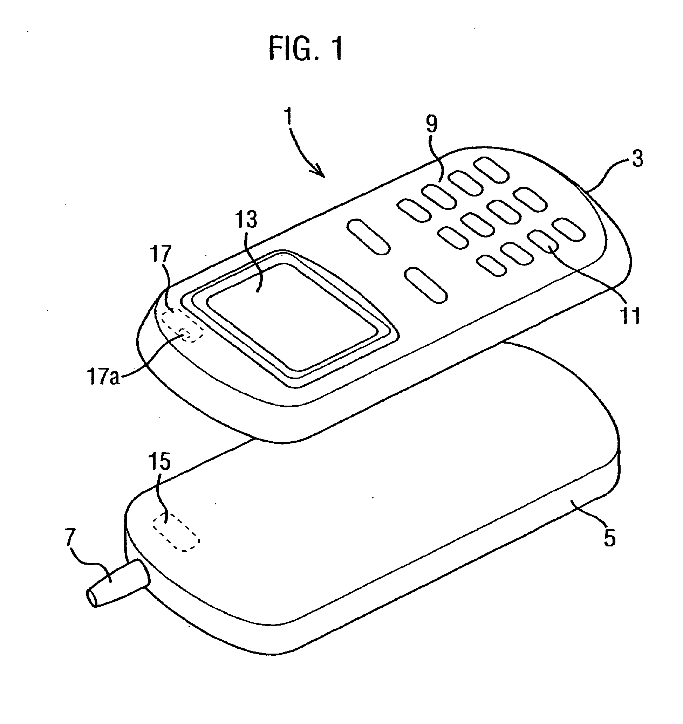

Figure 1 illustrates a mobile telephone or cellphone 1 comprising a main body 5 and an attachable, and in this case also removable, fascia 3. The fascia 3 is formed with a user input array 9 which comprises a plurality of holes 11 for engagement with key pads (not shown) provided on the main body 5 of the mobile telephone 1. The fascia 3 is also provided with, in this example, a transparent plastics window 13 through which a liquid crystal display (LCD) (not shown in Figure 1) provided on the main body 5 of the mobile telephone 1 can be viewed. The mobile telephone 1 also comprises an aerial 7 which allows telecommunication signals to be transmitted and received.

Mounted upon, or embedded within, an inside surface of the removable fascia 3 is a passive data storage device 17. Generally, the fascia 3 will be moulded from a plastics material and the passive data storage device 17 encapsulated in a housing fitted to (for example embedded in or mounted to the surface of) the fascia 3 during the moulding process.

The main body 5 of the mobile telephone 1 carries a reader unit 15. The reader unit 15 is positioned such that, when the fascia 3 is fitted to the main body 5, the passive data storage device 17 will be in range of, as shown will lie adjacent to, the reader unit 15 so that, as will be described in detail below, couplers of the passive data storage device 17 and reader unit 15 couple to enable the passive data storage device 17 to derive a power supply from a signal supplied by the reader unit 15 and, when so activated, to transmit control data contained in its memory.

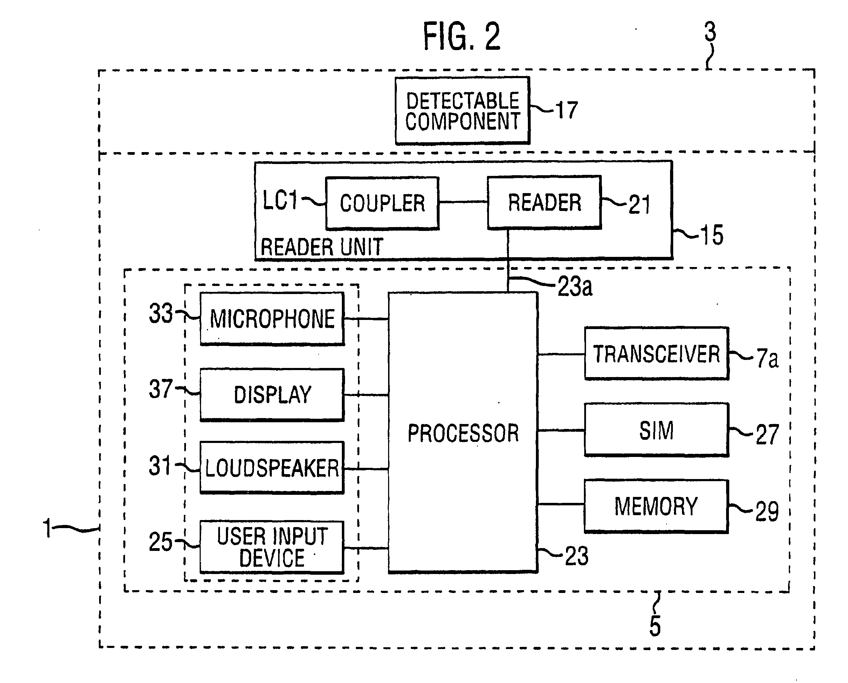

Figure 2 shows a functional block diagram of the mobile telephone 1. The main body 5 of the mobile telephone 1 comprises: a microphone 33 and a loudspeaker 31 enabling a user to input speech and hear audio output respectively; a user input device 25 (in this case a keyboard) enabling the user to input numbers to be called, other information and instructions for controlling various features of the mobile telephone 1; a display 37 on which incoming or outgoing telephone numbers, SMS text messages and other information can be displayed; and a transceiver 7a which with the aerial 7 shown in Figure 1 enables transmission and reception of communications over a mobile telecommunications network using, for example, the GSM (Global System for Mobile Communications), GPRS (General Packet Radio Service) or 3G (Third Generation, GSM) system, or future such networks.

The above functional components are coupled, via appropriate interfaces (not shown) to a mobile telephone processor 23 which controls the overall operation of the mobile telephone 1. The mobile telephone processor 23 is associated with a non-volatile memory 29 which has a read-only portion which contains control operation software data and information data and a writable portion that, for example, enables storage of telephone numbers and messages input to the mobile telephone by the user or received by the mobile telephone over the mobile telecommunications network.

As is known in the art, the mobile telephone 1 further comprises a subscriber identification module (SIM) 27. The SIM 27 is a detachable module which provides user specific data and also algorithms and data specific to the operator of the mobile telecommunications service provider. The mobile telephone processor 23 is coupled to the reader unit 15 via line 23a.

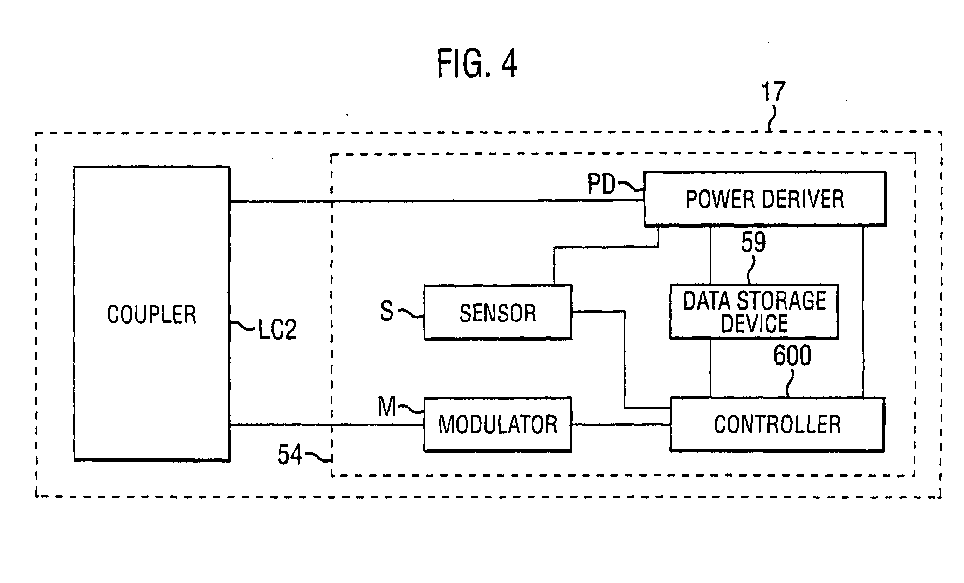

The reader unit 15 comprises a reader 21 which is coupled to the mobile telephone processor 23 via line 23a and which is also coupled to a first coupling element or coupler LC1 arranged to couple to a similar second coupling element LC2 (see Figure 4) carried by the passive data storage device 17 when the replacement fascia or cover portion 3 is fitted to the main body of the mobile telephone.

Although not shown in Figure 2, it will be appreciated that the components of the mobile telephone 1 and the reader unit 15 will be powered by the battery of the mobile telephone 1, although a separate power supply may be provided.

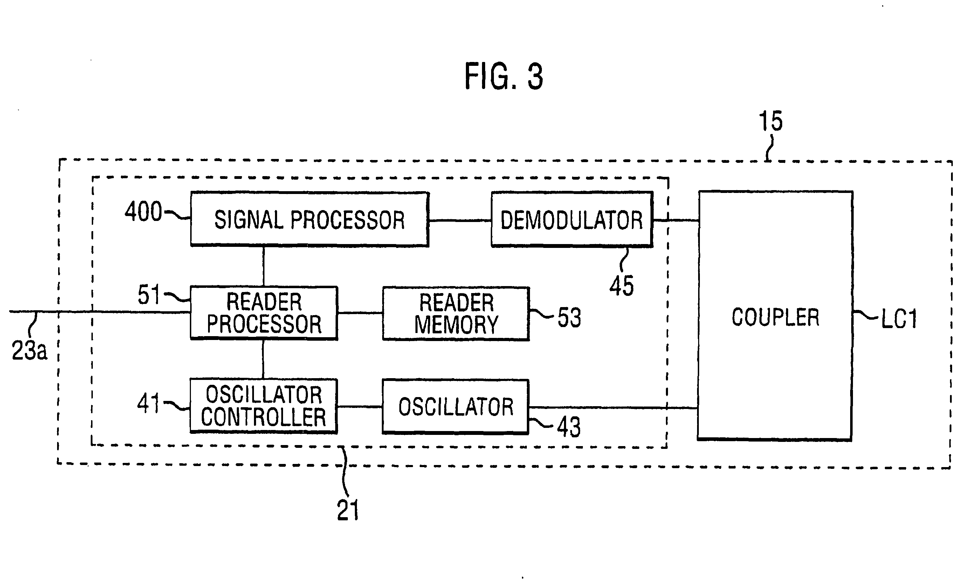

Figure 3 shows an embodiment of the reader unit 15 which consists of the coupling element or coupler LC1 and the reader 21. The reader 21 has a reader microprocessor or microcontroller 51 having a memory 53 which will generally be non-volatile but could be volatile if backed-up, for example battery backed-up. An oscillator 43 controlled by an oscillator controller 41 under the control of the reader microprocessor 51 supplies an oscillator output signal to the coupling element LC1. The reader microprocessor 51 controls the oscillator controller 41 to control activation of the oscillator 43 and, in this embodiment, also controls the oscillator control 41 to interrupt the output of the oscillator 43 to provide a signal from which the data storage device 17 within the fascia 3 can derive a clock signal as will be described below.

The first coupling element LC1 is also coupled to a demodulator 45 which enables the modulation to be recovered from an amplitude modulated signal. The recovered modulation is supplied to signal processor 400 which supplies a digital signal representing a string of binary ones and zeros to the reader microprocessor 51. In response to receipt of control data from the passive data storage device, the reader microprocessor 51 communicates with the mobile telephone processor 23 on data line 23a (see Figure 2) so as to affect the functionality or operating characteristics of the mobile telephone.

Figure 4 shows a simplified block diagram of one example of a passive data storage device forming the detectable component 17. As shown, the passive data storage device 17 comprises a passive data storage unit 54 and the coupler or coupling element LC2. The passive data storage unit 54 consists of a power deriver PD that derives a power supply for the passive data storage unit from the oscillator signal coupled from the reader unit 15 (shown in Figure 3) to the passive data storage device 17 by coupling of the first and second coupling element LC1 and LC2, a data store 59 in the form of a non-volatile memory storing control data for supply to the reader unit 15 and a controller 600 for controlling reading of data from the data store 59 and supply of that data to a modulator M that modulates the oscillating signal inductively coupled to the passive data storage device 17. Optionally, the passive data storage device may include a sensor S for reasons that will be set out below.

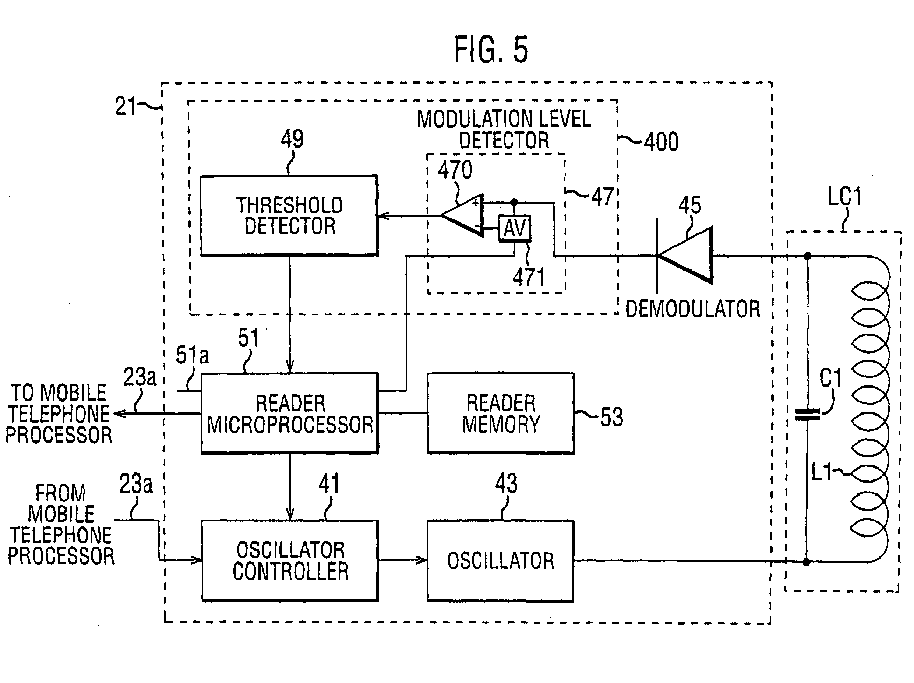

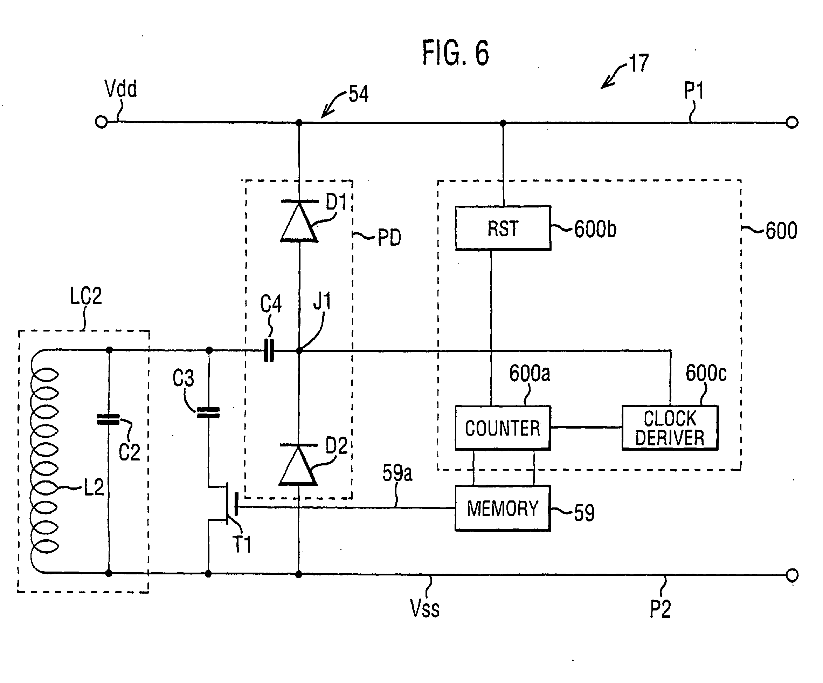

Figures 5 and 6 show, respectively, block diagrams illustrating one example of the reader unit 15 and one example of the passive data storage device 17. In this example, the passive data storage device 17 and reader unit 15 are adapted to communicate via an inductive coupling and each of the couplers LC1 and LC2 consists of a parallel connection of a capacitor and an inductor C1 and L1 and C2 and L2, respectively. The inductive couplers may form a tuned circuit, although this is not necessary at short range. The optional sensor S is not shown in Figure 6.

In the example shown in Figure 5, the demodulator 45 is a simple diode rectifier while the signal processor 400 consists of modulation level and threshold detectors 47 and 49. The modulation level detector 47 comprises a comparator 470 and an averaging circuit 471 controlled by the reader microprocessor 51. The output from the demodulator 45 is supplied to the positive input of the comparator 470. The average of the output of the demodulator 45 is supplied by the averaging circuit 471 to the negative or inverting input of the comparator 470.

The output of the comparator 470 is supplied to the threshold detector 49 which supplies to the reader microprocessor 51 either a one or a zero, depending upon the relationship between the signal and the threshold.

In this example, the oscillator controller 41 is a logic circuit or a transistor switch which controls the oscillator 43 to switch on or off the carrier signal to the first coupling element LC1 in accordance with a signal received from the reader microprocessor 51 and the averaging circuit 471 generally consists of an averaging capacitor connected between the inverted input of the comparator 470 and ground by a transistor switch or transmission gate which is controlled by the reader microprocessor 51 so as to be conducting while the carrier signal is present and after transients have settled but is off while the carrier is off and during carrier turn-on transients so that averaging is only carried out while there is a steady carrier signal.

As shown in Figure 6, the power deriver PD of the passive data storage unit 54 comprises series-connected diodes D1 and D2 and a capacitor C4 coupled between the coupling element LC2 and a junction J1 between the anode of the diode D1 and a cathode of the diode D2. The cathode of the first diode D1 is connected to a first power supply rail P1 (Vdd) while the anode of the second diode D2 is connected to a second power supply rail P2 (Vss). The capacitor C4 and the diodes D1 and D2 act effectively as a voltage doubler enabling the peak to peak voltage of a received AC or oscillating signal inductively coupled to the passive data storage device 17 by the coupling elements LC1 and LC2 to be used by the diodes D1 and D2 to derive a power supply for the passive data storage device 17 from the oscillating signal.

It will, of course, be appreciated that, in the interests of simplicity, the power supply connections to the remaining components of the passive data storage device 17 are not shown in Figure 6.

In this example, the controller 600 comprises a clock signal deriver 600c in the form of a missing pulse detector which is coupled to the junction J1 to derive a clock signal for the data storage unit 54 from the interrupted oscillator signal supplied by the oscillator 43 and an address counter 600a clocked by the clock signal. The controller 600 may also include a reset switch 600b to reset the counter 600a if the passive data storage device 17 is not powered for a predetermined time.

In this example, the controller 600 causes data to be read out from the data store 59 directly to the modulator M which comprises a series-connection of a FET T1 and a capacitor C3 coupled across the capacitor C2 of the coupler LC2. Thus, an output 59a of the data store 59 is coupled to the gate of the FET T1. In this example, the data store 59 is a serial read-only memory (ROM). It may, however, be any form of non-volatile memory that does not require battery backup such as a ROM, an EE-PROM (electrically erasable programmable ROM), a flash memory, F-RAM and so on.

When, as shown in Figure 1, the replaceable fascia 3 is fitted to the main body 5 of the mobile telephone 1 the coupling element LC1 of the passive data storage device 17 lies adjacent and in close proximity to the coupling element LC2 of the reader unit 15, inductively coupling the passive data storage device 17 to the reader unit 15. The oscillator 43 of the reader 21 generates a high or RF (radio frequency), typically 13.56MHz (MegaHertz), signal which is supplied to the first coupling element LC1 and inductively coupled to the passive data storage device 17 via the second coupling element LC2. The voltage doubler formed by capacitor C4 and diodes D1 and D2 thus derives a power supply for the passive data storage device and, when powered, the clock deriver 600c derives a clock signal from the interrupted oscillator signal and control data is output from the data store 59 on output 59a under the control of the counter 600a.

When control data is output by the data store 59 on the output line 59a, the data switches the FET T1. The loading across the inductor L2 varies in dependence upon whether the FET T1 is conducting or non-conducting, causing the oscillating signal to be modulated in accordance with the control data output from the data store 59.

The control data output from the data store 59 is extracted from the modulated oscillating signal by the demodulator 45, modulation level detector 47, threshold detector 49 and reader microprocessor 51 to provide a data input signal to the mobile telephone processor 23 (Figures 2 and 3) representing the data output from the data store 59.

The control data output from the data store 59 and downloaded by the reader microprocessor 51 (Figure 5) to the mobile telephone processor 23 (Figure 2) may then be stored in the writable portion of the memory 29. This control data may comprise at least one of software data, that is computer code executable or implementable by the mobile telephone processor 23, and information data that is stored in the memory 29 so as to be usable by the mobile telephone processor 23. As an example of software data, the data downloaded from the passive data storage device may include upgrades or modifications of the mobile telephone processor software where the fascia is supplied by or under licence from the manufacturer or supplier of the mobile telephone. Additionally or alternatively, software data may include updates or modifications to existing games software provided with the mobile telephone or new games software.

Information data may be provided as an alternative to or in addition to software data. Examples of information data that may be stored by the data store 59 include dialling or ringing tones and telephone numbers, Internet addresses and/or WAP addresses enabling, for example, the supplier of the fascia to attract buyers by supplying new ringing tones and/or to advertise itself or other companies by supplying their telephone numbers, Internet addresses or WAP addresses. Other examples of control data may include graphics data, audio data, image data, video data, biometric data, mobile telephone services, subscription services, network services, promotions, advertisements and so on.

As described above, the control data stored by the passive data storage device consists of the actual software and/or information data or the identity or address of the user's mobile network provider or another third party, service provider. As another possibility, the control data stored by the passive data storage device may be access data that enables the user to access such software and/or information data. For example, this access data may consist of a mobile telephone number or a WAP address that the user of the mobile telephone can contact to download new software data and/or information data or the identity or address of the user's mobile network provider or another, third party, service provider. As another possibility, downloading of access data may cause the mobile telephone processor 23 to make additional facilities for which the mobile telephone processor is already programmed but that are currently barred from the user available to the user, for example facilities such as international calling access, voicemail and message-taking facilities. As a further possibility, such access data may cause the mobile processor 23 to enable the user to access data previously stored in the memory 29 but not accessible to the user. Such data may include information data such as ringing tones, telephone numbers and/or WAP addresses and software data such as modifications to the processing software of the mobile telephone and/or upgrades or modifications to games software already available to the user or new games software.

The access data may be, for example, an identity code that enables the mobile telephone processor 23 to identify the particular type of fascia 3 by comparison with data stored in its memory. The mobile telephone processor 23 may then control the mobile telephone functionality and capability available to the user in accordance with the identified fascia 3.

The mobile telephone processor may be programmed so that, if it receives no control data or receives incorrect or non-understandable control data (for example if it receives the wrong access or identity code data), then the mobile telephone processor 23 may determine that the fascia is a counterfeit or unrecognised fascia and may modify or restrict the functionality of the mobile telephone. The mobile telephone processor 23 may inhibit access to all or certain optional features or functions and may continue to inhibit use of those optional features or functions until a genuine fascia is fitted to the mobile telephone. For example, if the mobile telephone processor 23 determines that the fascia is a counterfeit fascia, then the mobile telephone processor 23 may simply limit the number of functions available via the mobile telephone, for example the mobile telephone processor 23 may limit the available number of ringing or dialling tones or message taking options, for example, or may "lock" the mobile telephone so that only emergency outgoing calls may be made.

The mobile telephone processor 23 may also display a message to the user to alert them to the fact that the replacement fascia is not genuine. Where the use of a non-genuine replacement fascia is not detrimental to the functioning of the mobile telephone and does not potentially detrimentally affect the safety of the user, then the mobile telephone processor 23 may simply display a message to the user on the display 37 to alert the user to the fact that the fascia is not genuine.

The mobile telephone processor may also be programmed to enable it to identify genuine replacement fascias for other models of mobile telephone and to cause the display 37 to display to the user a message alerting them to the fact that the incorrect replacement fascia has been fitted if the mobile telephone processor determines that incorrect identity data has been received.

As described above, the passive data storage device is powered and so supplies its stored data to the reader unit 15 whenever the first and second coupling elements LC1 and LC2 are inductively coupled. The reader microprocessor may send the received data continually or periodically to the mobile telephone processor 23. Continually powering the passive data storage device 17 may, however, present a drain on the battery of the mobile telephone. Accordingly, the reader microprocessor 51 may be programmed to cause the oscillator control 41 to switch on the oscillator 43 only at predetermined intervals so that the passive data storage device 17 is periodically activated to send its data. Generally, it will be desirable for the passive data storage device 17 to transmit its data to the reader unit 15 only when a replacement fascia 3 has just been coupled to the main body 5 of the mobile telephone. This may be achieved by using a simple interlock system which provides a signal to the reader microprocessor 51 indicating whether or not a fascia 3 is present. As an example, part of the encapsulating housing of the passive data storage device 17 may carry a conductive strip (17a in Figure 1) that, when the fascia 3 is coupled to the main body 5, couples a pin (51a in Figure 5) of the reader microprocessor 51 to ground or earth and the reader microprocessor may periodically check the status of that pin. When the reader microprocessor 51 detects that the voltage at the pin 51a has gone high then it determines that the fascia 3 has been removed and when it detects that the voltage at that pin has again gone low it determines that either the fascia 3 has been refitted to the main body 5 or a different fascia 3 has been fitted and so activates the oscillator control 41 to switch on the power oscillator 43 to power up the passive data storage device 17. In this way, the passive data storage device 17 will be powered up to transmit its data only when a replacement fascia 3 or cover portion is fitted to the main body 5.

In the above described embodiments, the passive data storage device 17 is a synchronous passive data storage device, that is the clock signal of the passive data storage device 17 is controlled by and so synchronised with the reader microprocessor 51. However, the passive data storage device 17 may be an asynchronous device, that is the clock deriver 600c coupled to the junction J1 shown in Figure 4 may be replaced by a clock signal generator which is powered up when a power supply is derived by the passive data storage device to generate an independent clock signal for the passive data storage device. Also, although Figure 6 shows the use of a counter 600a to count out data from the data store 59 it will, of course, be appreciated that the data store 59 may be arranged simply to output its data an address at a time and that the counter 600a may not be necessary. In addition, the passive data storage device 17 may incorporate a microcontroller that controls read out of data from the data store 59 which may enable different areas of the data store 59 (and so different data) to be accessed and read out in accordance with instructions supplied by the reader microprocessor 51. Such instructions may be transmitted by representing 0 and 1 as long and short duration interruptions of the oscillator signal.

In the above described embodiments, the passive data storage device modulates the signal received from the reader by, under control of the counter 600a and clock divider 600c, reading data out of the data store 59.

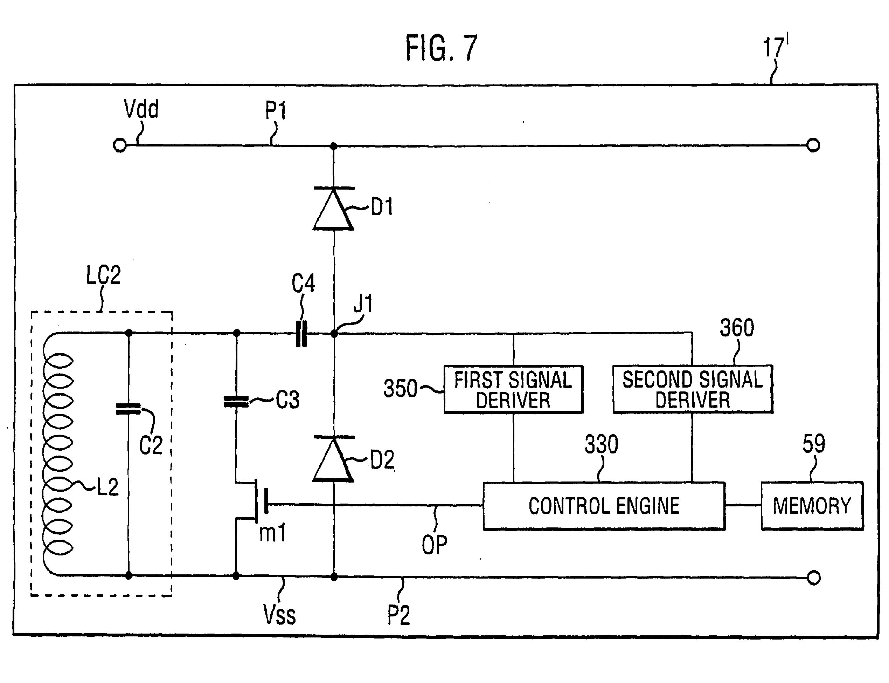

Figure 7 shows an example of another passive data storage device 17' that may be used in place of the passive data storage device 17 shown in Figure 6. This data storage device uses a different controller from that shown in Figure 6. In this case, the controller comprises a control engine 330 arranged to recognise a number of different codes (in the form of predetermined sequences of ones and zeros) transmitted to it by the reader microprocessor 51. In this example, the reader microprocessor 51 is arranged to transmit ones and zeros by causing the oscillator control 41 to interrupt the output of the oscillator 43 for short and long durations, respectively.

In this embodiment, a clock signal for the control engine 330 is derived from the signal supplied by the oscillator 43 by a first signal deriver 350 in the form of a fast missing pulse detector coupled to junction J1 while the control engine 330 is arranged to extract the data transmitted by the reader 17' using the output of the first signal deriver 350 and the output of a second signal deriver 360 in the form of a slow missing pulse detector also coupled to the junction J1.

The timeout periods of the fast and slow missing pulse detectors are set so that the output of the fast missing pulse detector will have one of two widths dependent upon whether the particular data bit is a binary "zero" or a binary "one" while the slow missing pulse detector will provide a pulse only when a particular data bit is a binary "one". The control engine 330 can thus determine from the outputs of the first and second signal derivers 350 and 360 whether a received bit is a binary "zero" or a binary "one". As set out above, the control engine 330 is programmed to enter a number of different states dependent upon the instruction code received from the reader unit 15. These different states may cause the control engine to read out data from different areas of the data store 59. The data store 59 may, however, be an electrically erasable memory in which case at least one of the states of the control engine 330 may enable the control engine to erase the data in a portion of the data store 59 and to write new data supplied by the reader unit 15 into the data store 59. This would enable, for example, the reader unit 15 to overwrite at least a part of the content of the data store 59 after the fascia 3 has first been coupled to the main body 5 so as to limit or inhibit access to the functionality provided by the data stored in the data store 59 if the fascia is subsequently removed and fitted to a different mobile telephone. As another possibility, the mobile telephone processor may be programmed to place a time limit on the availability of the additional functions provided by the data stored in the data store 59.

Although in the above described embodiment, the control engine 330 is a state machine with its own non-volatile memory it will, of course, be appreciated that the state machine may be replaced by an appropriately programmed microprocessor or microcontroller also having its own memory. Also, different methods for communicating instructions from the reader unit 15 to the control engine 330 may be used.

Where, as described above, the data store 59 is writable, then it may also be used to extend the memory capacity of the mobile telephone to store further telephone numbers and/or other data. The passive storage device 17 may also be used to provide a record of historical degree and frequency of use of the mobile telephone. Further details of the writing and reading operations that may be carried out by the control engine 330 are to be found in UK Patent Application number: 0031518.4 or the corresponding PCT application number GB01/05690.

Data may also be written to the mobile telephone SIM card by the reader unit and mobile telephone processor.

In the above described embodiments, the actual components of the user interface keypads or keyboard are carried by the main body of the mobile telephone and the fascia 3 simply provides apertures through which the keypads extend.

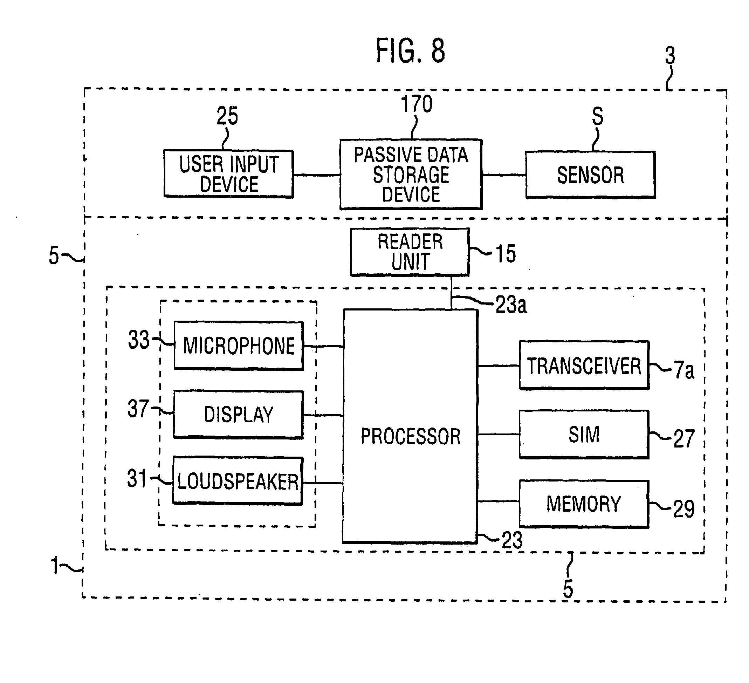

Figure 8 shows a functional block diagram, similar to Figure 2, of another example of a mobile telephone in accordance with the present invention. This mobile telephone differs from that shown in Figure 2 in that the user input device 25, in this example the keyboard, is provided within the fascia 3 and is arranged to communicate with a passive data storage device 170 that, when the fascia 3 is fitted to the main body 5 of the mobile telephone, derives a power supply from the signal supplied by the reader oscillator 43 as described above and, in response to input by the user to the user input device 25, communicates this input to the reader unit 15 by modulating the oscillator signal in a manner similar to that described above. In this case, the fascia may include an optional sensor S coupled to the passive data storage device.

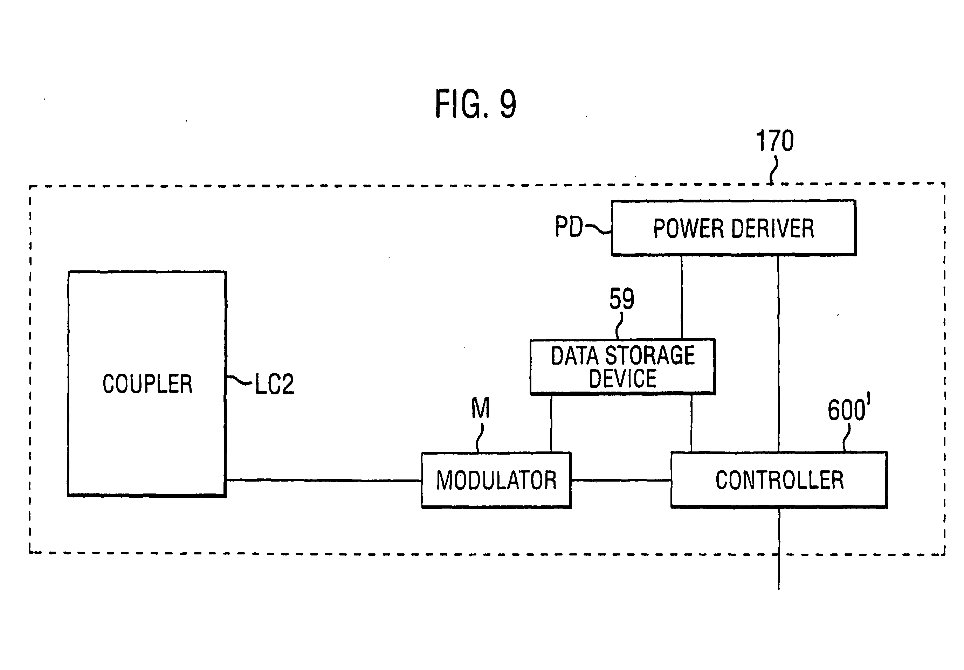

Figure 9 shows a functional block diagram of the passive device 170. This passive data storage device is similar to that shown in Figure 7 with the controller 600' having the configuration described above with reference to Figure 7 (that is it will include a control engine in the form of a state machine, microprocessor or microcontroller with its own memory). The passive data storage device 170 differs from that shown in Figure 7 in that the controller 600' incorporates a keypad or keyboard scanner (either by programming the controller or possibly by providing a dedicated user input interface) to enable it to communicate with the user input device 25 shown in Figure 8 and, in response to keystrokes made by the user of the user input device 25, to communicate to the reader unit 15 shown in Figure 8 code data (a predefined sequence of zeros and ones) that will identify to the mobile telephone microprocessor 23 the key that has been activated or depressed by the user. The passive data storage device 170 communicates this data in the same manner as described above, that is by the controller 600' causing the modulator M to modulate the oscillator signal supplied by the reader unit 15 in accordance with the data to be transmitted.

Any known form of code for identifying the keypads may be used, for example, the ASCII code system may be used. The data store 59 stores control data of the type described above, that is software data and/or information data that affects the functionality of the mobile telephone. In this case, the controller 600' of the passive data storage device 170 is programmed so as to download or communicate to the reader unit 15 control data from the data storage device 170 when the fascia is first fitted or first refitted to the main body 5 of the mobile telephone 1 and then subsequently to monitor keypad activation by the user and to communicate keystrokes input by the user to the reader unit 15 as described above.

Placing the entirety of the keyboard in the fascia 3 means that it is not necessary for the interior shell of the main body 5 of the mobile telephone to be riddled with holes corresponding to the keypad (which reduces the efficacy of any electromagnetic interference (EMI) shielding). Rather, the manufacturer of the mobile telephone should be able to use a continuous shielding layer, apart from coupling connections required to connect to the display.

Providing the entirety of the user input device 25 within the fascia 3 means that the mobile telephone may be fitted with different fascias for different applications, for example, a mobile telephone fascia that facilitates use as a mobile telephone and a games fascia that facilitates playing of a game.

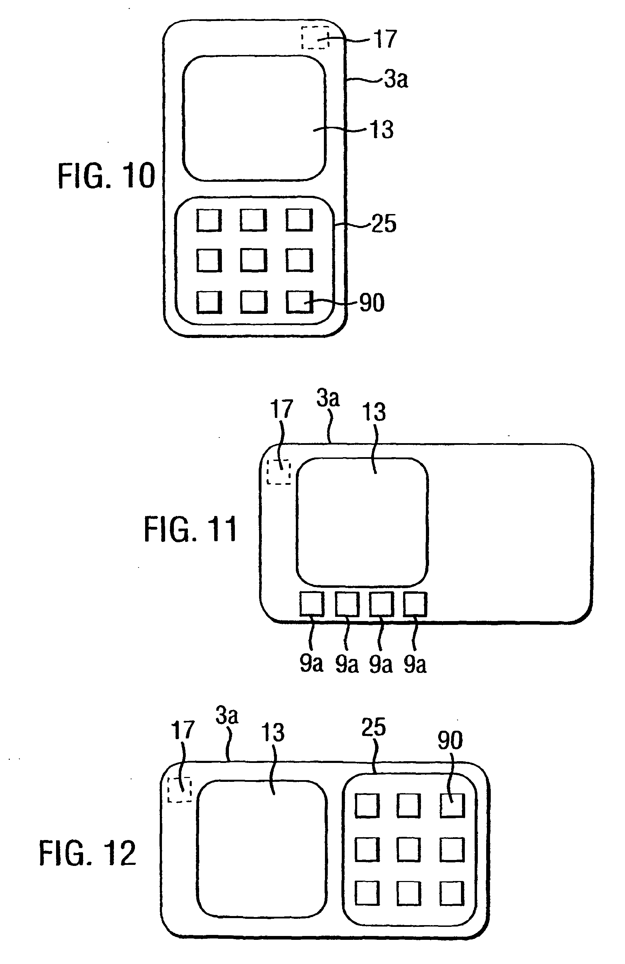

Figures 10 and 11 illustrate schematically examples of the front surfaces of two such fascias 3a and 3b. In each case, the display window 13 has the same size and dimension but in Figure 11 the display window 13 and user input device 25 are arranged so that the display is viewed in a landscape orientation while in Figure 10 the display is viewed in a portrait orientation.

The fascia 3a shown in Figure 10 has a keyboard 25 with a keypad 90 arrangement similar to that indicated by Figure 1.

In the fascia 3b shown in Figure 11, the keyboard 25 is replaced by games key inputs 9a each of which corresponds to a gaming control function, analogous to those present on a computer games console.

Separating the user input device 25 from the main body 5 of the mobile telephone enables the position, type, number and size of the keys 9a, 90 to be varied without affecting the functionality of the main body of the mobile telephone. All that is required is that the mobile telephone processor can identify from control data downloaded from the passive data storage device 170 when the fascia is first fitted, the type of user input device 25 and can then respond to the key stroke data code communicated to it from the passive data storage device 170. The control data also enables the mobile telephone processor to determine the use orientation of the display and to control the LCD accordingly.

This enables, as will be appreciated from Figures 10 and 11, the user to attach to the main body 5 a fascia carrying a user input device appropriate to the task that he wishes to carry out. Thus, as described above, a dedicated games fascia may be provided. In addition to providing a different keypad layout, the keypads themselves may be differently constructed so that, for example, soft more reactive keypads may be provided in the games fascia to allow better, faster game play. In addition, it may also be possible to build a simple joystick into the games fascia to facilitate games play.

In addition to telephone and games applications, where the mobile telephone also doubles as a personal digital assistant (PDA) having, for example, word processing and/or spreadsheet facilities, then different fascias may be provided for these options so that, for example, instead of a mobile telephone type keypad, a fascia adapted for word processing may carry, providing sufficient space is available, a full QWERTY type keyboard.

As another possibility, where the weight of the mobile telephone or PDA is a consideration or only limited functions are required, for example whilst the user is on holiday, then a small minimalistic user input device 25 with a limited number of keypads may be provided. Also, where the user wishes to lend the mobile telephone to a younger family member, then fascias having a "restricted use" user input device may be fitted that restricts the functions of the mobile telephone that the younger family member can access.

In addition, the passive data storage device may store "macros" relating to certain keys allowing, for example, high speed input of words that relate to that particular fascia. Thus, for example, a fascia designed for use by football supporters may have words such as "goal", "net", "idiot!" and so on associated with keys of the fascia while a business fascia may have a passive data storage device that stores macros for phrases such as "call you later", "meet me at", "time", in each case facilitating, for example, the sending of text messages. In each case, because the user input device is independent of the main body or mother unit of the mobile telephone, the actual keypads may carry visual identifiers identifying the macros with which they are associated. In addition, the passive data storage device may contain as part of the control data, spell checkers, crosswords, word translators, dictionaries etc facilitating use of the mobile telephone as an educational tool.

As will be appreciated, because the user input interface device is independent of the main body 5 of the telephone, specialist keypads that enable greater levels of touch or pressure sensitivity may be provided and the controller 600' of the passive data storage device programmed to respond to different degrees of touch or pressure in a predetermined manner enabling, for example, different types of characters for example, upper, lower, bold, italics and so on, to be generated dependent on how hard the key is pressed. Such a fascia may also be specifically adapted to facilitate drawings of graphics with different keys controlling drawings of lines in different directions with the extent or thickness of the line being determined by the user pressure on the keypad or even some of the keypads being associated with simple graphical shapes such as circles, squares etc. The passive data storage device and mobile telephone processor may also be programmed to communicate data relating to musical notes and, for example, a dedicated fascia be may provided that enables the user to create tunes using different levels of pressure of touch to change the note or key, for example. Such a fascia may also be specifically adapted to enable its use by a person with a disability. For example, the fascia may be specifically adapted for use by a person having, for example a physical, visual, hearing or mental disability. As examples: a fascia may be specifically adapted for use by a visually impaired or blind person by providing the user input with specific tactile features; a fascia may be specifically adapted for use by a hearing impaired or deaf person by providing the user input with a speech-to-text facility that enables a telephone text mode; a simplified fascia may be provided with symbols and icons for people with learning difficulties; and a fascia may be provided that enables single switch operation by scanning for people with physical disabilities. As a further possibility, a fascia may provide a link to a telephone relay service such as "Typetalk" for use by hearing impaired or deaf people.

In the above described examples, separate fascias carrying the user input device are provided for different functions. As another example, the passive data storage device may contain an orientation sensor (shown as sensor S in Figures 4 and 8) that enables the controller of the passive storage device to determine the orientation of the mobile telephone and thus of the fascia so that, when the mobile telephone is in the orientation shown in Figure 10, the passive data storage device instructs the mobile telephone processor to control the display in the portrait orientation while when the mobile telephone is in the orientation shown in Figure 12, the passive data storage device instructs the mobile telephone processor to control the LCD display in landscape orientation, facilitating its use for graphics and games play. In this case, the user input device 25 itself would be physically identical in both orientations but the control signals supplied by the controller of the passive data storage device to the main body 5 of the mobile telephone in accordance with keystrokes made by the user will depend upon the orientation of the fascia.

As another possibility instead of using an orientation sensor, the fascia may carry a user input, for example one of the keypads that, when depressed, instructs the controller of the passive data storage device to communicate with the mobile telephone processor so that the orientation of the display is switched from portrait to landscape or vice versa.

The optional sensor S shown in Figures 4 and 8 need not necessarily be an orientation sensor but could be, for example, an environmental sensor sensitive to any one or more of temperature, humidity, sound, odour, a fluid, a gas, water, magnetic field, radio frequency, infra red, vibration, a chemical, pressure, for example, e.g. a barometrics sensor, orientation, position, height, or light that provides, via the first and second couplers, a signal regarding the environmental conditions to the reader unit that affects the overall operation of the mobile communications device. For example, where the sensor is a light sensor, then the reader unit may be supplied with a low light signal that automatically causes the processor of the mobile communications device to activate a back light or where the sensor senses noise level the reader unit may cause the mobile telephone audio volume to be raised or lowered depending upon the ambient noise sensed by the sensor.

In the above described embodiments, the reader unit 15 is, as shown in Figure 2, separate from the main components of the mobile telephone. The reader unit 15 may, however, be provided within the main components of the mobile telephone. For example, the reader unit may be provided in the SIM card so that it is not necessary to adapt the reader unit for different hardware.

In the above described examples, the user input device 25 carried by the fascia is the keyboard or key input. It may, however, also be possible to incorporate into the fascia 3a or 3b within the window 13 a substantially transparent touch sensitive array such as a capacitive array that enables a user to input commands using their fingers or a stylus. In this case, the keyboard may be omitted and the display may display soft keys that are activatable by the user using the stylus or a finger. The mobile telephone processor may then be programmed to display different types of soft keys when the fascia is used in the landscape rather than the portrait orientation and, of course, different types of soft keys may be provided on different types of fascias.

In the above described examples, the user input device and passive data storage device 17 are provided in a fascia for the mobile telephone. This need not necessarily be the case. The mobile telephone may have a conventional fascia or a fascia such as that shown in Figure 1 but without the passive data storage device and a separate user interface device having a passive data storage device and the functional components shown in Figure 9 may be provided so that, when the separate user interface device is coupled to the mobile telephone by a mechanical coupling mechanism or is positioned sufficiently close to the mobile telephone that the first and second couplers LC1 and LC2 are inductively coupled, then the controller of the passive data storage device causes control data to be read from the data storage device so as to modulate the oscillating signal to communicate to the mobile telephone instructions to the mobile telephone processor that it is to ignore the in-built user interface and to receive user input instructions from the user input device. This would enable, for example, a user to have a separate lightweight, non-self-powered user interface device having as the user interface device at least one of a keypad, keyboard, touch screen, joystick, finger print device, and digitizing tablet that enables the user, by bringing the separate user interface device into contact with or in proximity to the mobile telephone, to override the normal user interface of the telephone and to use the user interface device to facilitate, for example, playing of games software or, where the user interface device enables a QWERTY type keyboard layout, to facilitate entering of text messages or notes. It may also be possible to provide the display in the fascia.

In the above described examples, data carried by the passive data storage device affects the functionality or capabilities of the mobile telephone. The reader processor may be programmed so as to automatically cause the mobile telephone processor to switch the mobile telephone from a standby mode to an active mode only when the fascia is correctly fitted to the main body, thereby preventing any accidental operation of the mobile telephone with its fascia removed.

Some examples of mobile telephones, in particular those incorporating PDA like facilities, include a movable, for example hinged, cover or lid. In this case, it will be appreciated that, in order for the passive data storage device to communicate with the reader unit when the cover is in the open, working condition, the passive data storage device should be appropriately located in the cover portion so as to be close to the reader unit when the cover or lid is in the open condition. As another possibility or additionally, a passive data storage device may be located in a part of the cover portion, for example, remote from a hinge, such that the couplers LC1 and LC2 are only coupled when the cover or lid is closed and the mobile telephone processor may be programmed to move from a standby to a full active mode when the reader unit provides a signal indicating that the first and second couplers LC1 and LC2 are no longer coupled.

In the above described embodiments, the passive data storage device 17 uses amplitude modulation to transmit data to the reader unit 15. It will, however, be appreciated that frequency modulation may be used as may phase modulation as described in WO 97/23060 (PCT/GB96/02975).

In the above described embodiments, the first and second coupling elements are arranged to couple inductively. The first and second coupling elements may be arranged to couple in any other manner that requires the first and second coupling elements to be in close proximity but not in physical contact with one another. For example, the first and second coupling elements may be arranged to couple capacitively. These non contact arrangements have advantages over ohmic coupling arrangements because the use of ohmic coupling has the disadvantage that actual electrical contact needs to be established between the first and second coupling elements and that this requires the coupling elements to be exposed which may make manufacture of the fascia 3 more difficult and repeated removal and fitting of fascias may cause wear and tear of the coupling elements. Also, both capacitive and ohmic coupling require more accurate alignment and closer positioning of the first and second coupling elements than inductive coupling. Other forms of coupling, such as an optical coupling, may also be used.

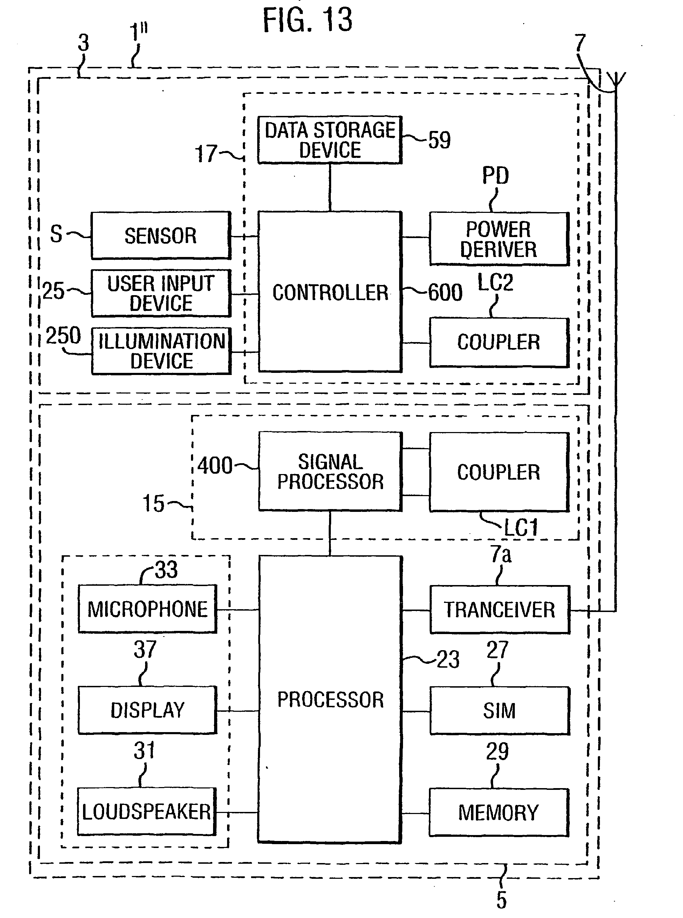

Figure 13 shows a functional block diagram of a mobile telephone 1" wherein the oscillator and oscillator control are omitted from the reader unit and the couplers LC1 and LC2 are tuned to the RF signal emitted by the antenna 7 of the mobile telephone so that the passive data storage device 17 derives its power supply from the RF signal transmitted by the mobile telephone and data communication between the passive data storage device 17 and the reader unit is achieved in the manner described above with reference to Figures 6 and 7 by varying the loading of the inductive coupler LC2 to provide an amplitude modulated signal.

When powered-up, the passive data storage device 17 transmits data stored in the data store 59 by modulating the RF signal. The positioning of the reader unit 15 on the main body of the mobile telephone and the positioning of the passive data storage device 17 on the facia 3 are such that the coupler LC1 is positioned very close to (within 8mm of) the coupler LC2 and accordingly will pick up the modulation from the passive data storage device 17. This enables the passive data storage device to communicate its identity code to the processor 23 of the mobile telephone 1" upon powering-up of the mobile telephony RF carrier signal. The coupler LC2 may be positioned between the antenna 7 and the coupler LC1 to shadow or hide the coupler LC1 from the RF signal supplied by the antenna so that the signal supplied by the data storage device is not swamped by the antenna signal.

The processor 23 of the mobile telephone may be programmed to expect an identity code from the signal processor 400 of the reader unit 15 upon power-up so that there is no need for any handshaking operation.

The controller 600 of the passive data storage device may be programmed to transmit its identify code a number of times, for example three times, and then to cease modulation of the RF signal transmitted by the mobile telephone 1" until, after the mobile telephone has ceased transmitting, the mobile telephone next commences transmission of its RF signal.

The processor 23 of the mobile telephone may be programmed to send a clean, unmodulated, RF signal for a few milliseconds before commencing frequency modulation in accordance with the GSM standard so that the RF signal that the passive data storage device modulates is clean, that is unmodulated. This should, however, not be necessary where the passive data storage device uses amplitude modulation to convey its data to the reader unit 15.

The passive data storage device may comprise an application specific integrated circuit (ASIC) with the data store 59 comprising, for example, an electrically erasable memory, enabling the keyboard scanner software to be configured at factory level to co-operate with the particular type of keypad or keyboard carried by the particular cover into which the passive data storage device is to be incorporated.

The embodiments described with reference to Figures 2 to 12 may be modified so that the mobile phone processor 23 carries out the functions of the reader processor 51 so that, the reader unit 15 need not include the reader processor 51 and reader memory.

As another possibility coupling between the passive data storage device 17 and the reader unit 15 may be via a coupling arrangement such as described in the applicants co-pending published International Application No. WO00/31676 may be used .

The present invention may be applied to computing devices such as personal computers, PDA (personal digital assistant) or games console with facilities for enabling communication over a mobile telecommunications network. The control data may comprise software data in the form of new software and/or upgrades for existing software provided on the PDA including, for example, games software, word processing, spread sheet and other applications software. The control data may alternatively or additionally include information data such as, for example, tunes and images that may be played via a loudspeaker or shown on the a display under control of the processor of the computing device. As described above, the control data may alternatively be in the form of access data that enables the reader unit to instruct the processor where to obtain data represented by the access data by, for example, accessing a particular site on the Internet or another network using the communications interface. Such data may be pre-stored in a memory, or available via a communications interface.

The present invention may be applied to mobile communications devices having a microprocessor or microcontroller where a portion or the whole of the housing or a cover of the device is replaceable or where an attachable component such as a tool or a plug-in key or component can be attached to or fitted into or onto the device.

Mobile communication devices having reader units as described above may also communicate with one another. Communication by RF or similar electromagnetic frequency has advantages over more sophisticated technology such as BlueTooth™ technology in that the RF communication system is cheaper and is shorter range so that there is less possibility of interference or crosstalk with other devices. Typically a reader unit will be able to communicate with a data storage device or other reader unit over a range of up to about six inches. Also, in contrast to BlueTooth™ which uses a frequency of about 2.4 GigaHertz, uses frequency hopping and a large number, typically 82, channels, RF communication uses a fixed single frequency, typically 13.56 MegaHertz, which provides a continuous signal which is modulated, for example amplitude modulated, by the data to be transmitted. In addition, unlike BlueTooth™, such communications do not require the use of an external protocol. All that is required is that the reader unit can demodulate the modulated carrier signal supplied by the data storage device.

Such a mobile communications device incorporating a reader unit may be used in many forms of commerce, as a payment method, to obtain account details and so on.

## Claims

1. A portable communications device comprising a first body portion (5) and a second body portion (3), wherein:
the first body portion (5) has a transceiver for enabling transmission and reception of communications over a mobile telecommunications network, first control means (23, 51) for controlling operation of the device, and signal supplying means (41, 43 or 7a) for supplying a carrier signal;
the first body portion (5) and the second body portion (3) have respective first and second wireless coupling means (LC1 and LC2) arranged in close proximity to one another to enable wireless coupling of the carrier signal from the first wireless coupling means (LC1) to the second wireless coupling means (LC2);
the second body portion (3) carries a data storage device (17) having a memory (59) storing data, power supply deriving means (PD) for deriving a power supply from the carrier signal coupled to the second wireless coupling means (LC2), second control means (600, 600') for reading data from the memory (59) in response to derivation of a power supply by the power supply deriving means (PD), and modulating means (M) for modulating the carrier signal in accordance with data read from the memory (59);
the first body portion (5) has data extraction means (45) for extracting data from the modulation of the carrier signal and data supplying means (400) for supplying the data to the first control means (23, 51) to affect a function relating to the operation of the portable communications device.

2. A device according to claim 1, wherein the signal supplying means (41, 43) is separate from the transceiver (7a).

3. A device according to claim 1, wherein the signal supplying means (7a) comprises a radio frequency carrier signal supplying means comprising a carrier signal generator of the transceiver.

4. A device according to any of the preceding claims, wherein the second body portion (3) is removable.

5. A device according to any of the preceding claims, wherein the coupling between the first and second coupling means (LC 1 and LC2) is inductive or capacitive.

6. A device according to any of the preceding claims, wherein the signal supplying means (41, 43) includes signal interrupting means for periodically varying the signal and the data storage device (17) has clock signal deriving means (600, 600') for deriving a clock signal for the data storage device (17) on the basis of the periodic variations.

7. A device according to any of claims 1 to 5, wherein the data storage device (17) has clock signal generating means for generating a clock signal for the data storage device (17) in response to derivation of a power supply by the power supply deriving means.

8. A device according to claim 7, wherein the clock signal generating means comprises a missing pulse detector.

9. A device according to any one of the preceding claims, wherein the memory (59) is a writeable memory (59), the signal supplying means (41, 43) includes data supplying means (400) for modulating the carrier signal in accordance with data to be written to the memory (59) and the data storage device (17) has write means (360, 330) for deriving the data from the modulated carrier signal and writing the derived data into the memory (59).

10. A device according to claim 9, wherein the first control means (23, 51) has use information deriving means for obtaining information regarding use of the portable communications device and is operable to cause the carrier signal to be modulated in accordance with the use data so as to enable the write means (360, 330) to write the use data into the memory (59).

11. A device according to any one of the preceding claims, wherein the first control means (23, 51) is operable to cause the signal supplying means (41, 43) to supply the carrier signal at a predetermined time such as on powering up of the device, when the second body portion (3) is first coupled to the first body portion (5) or at predetermined intervals.

12. A device according to any of the preceding claims, wherein the data stored in the memory (59) comprises at least one of: software data comprising at least one of operation software data for the portable communications device and games software data, identity data, access data for enabling the first control means (23, 51) to access operation software data or games software data, identity data and information data comprising at least one of graphics data, audio data, image data, video data and text data.

13. A device according to any of the preceding claims, wherein user operation means (25) is associated with the second body portion (3).

14. A device according to any of claims 1 to 8, wherein user operation means (25) is carried by the second body portion (3) and the data storage device (17) is operable, in response to user input via the user operation means (25), to supply input data regarding user operation of the user operation means (25) to the first body portion (5).

15. A device according to claim 14, wherein the data storage device (17) is operable to supply instructions to the first control means (23, 51) to configure a display (13) of the portable communications device for use in one of a number different orientations.

16. A device according to claim 13, 14 or 15, wherein the user operation means (25) comprises at least one of a keyboard, a key pad, a touch screen, a joystick, a digitizing table or other input.

17. A device according to any of claims 13 to 16, further comprising a separate user interface device having a different form of configuration of user operation means, the user interface device also having a passive data storage device, power deriving means for deriving a power supply when coupling means of the user interface device are coupled to coupling means of the portable communications device and data supplying means for supplying, in response to operation by a user of the user input device, user input data to the first control means (23, 51) when the user interface device is coupled to the portable communications device.

18. A device according to any of the preceding claims, wherein the data stored in the memory (59) comprises data enabling the first control means (23, 51) to identify the second body portion (3) and the first control means (23, 51) is operable to inhibit at least one function or operating characteristic of the portable communications device if the first control means (23, 51) does not receive identification data or does not receive correct identification data.

19. A device according to any of the preceding claims, wherein the or a second body portion (3) carries an orientation sensor and the second control means is operable, in response to an orientation signal from the orientation sensor to communicate to the first control means (23, 51) data regarding the orientation of the device to cause the first control means (23, 51) to control the orientation of a display screen accordingly.

20. A device according to any of the preceding claims, wherein the second body portion (3) carries a display of the device.

21. A device according to any of the preceding claims, wherein the or a second body portion (3) carries, instead of or in addition to the data storage device (17), at least one environmental sensor responsive, for example, to at least one of temperature, pressure, orientation, position, height, humidity, light, sound, a chemical, a fluid, water, magnetic field, an odour and the data storage device (17) is operable to communicate to the first control means (23, 51) data relating to an output of the sensor to affect an operation of the device.

22. A device according to any one of the preceding claims, wherein the second body portion (3) comprises a fascia of the device.

23. A device according to any one of the preceding claims, wherein the first body portion (5) comprises a main body portion and the second body portion (3) comprises a housing portion of or an accessory for the main body portion.

## Patentansprüche

1. Tragbares Kommunikationsgerät, umfassend ein erstes Körperteil (5) und ein zweites Körperteil (3), wobei
das erste Körperteil (5) einen Sendeempfänger zum Senden und Empfangen von Kommunikationen über ein Mobilfunknetz, ein erstes Steuermittel (23, 51) zum Steuern des Betriebs des Geräts und ein Signalliefermittel (41, 43 oder 7a) zum Liefern eines Trägersignals aufweist;
das erste Körperteil (5) und das zweite Körperteil (3) jeweils erste und zweite drahtlose Kopplungsmittel (LC1 und LC2) aufweisen, die nahe beieinander angeordnet sind, um das drahtlose Koppeln des Trägersignals vom ersten drahtlosen Kopplungsmittel (LC1) an das zweite drahtlose Kopplungsmittel (LC2) zu ermöglichen;
das zweite Körperteil (3) eine Datenspeichervorrichtung (17) mit einem Speicher (59) zum Speichern von Daten, ein Stromzufuhrsableitungsmittel (PD) zum Ableiten einer Stromzufuhr aus dem an das zweite drahtlose Kopplungsmittel (LC2) gekoppelten Trägersignal, ein zweites Steuermittel (600, 600') zum Lesen von Daten aus dem Speicher (59) in Antwort auf das Ableiten einer Stromzufuhr durch das Stromzufuhrableitungsmittel (PD) und ein Modulationsmittel (M) zum Modulieren des Trägersignals gemäß der aus dem Speicher (59) gelesenen Daten trägt;
das erste Körperteil (5) ein Datenextraktionsmittel (45) zum Extrahieren von Daten aus der Modulation des Trägersignals und ein Datenliefermittel (400) zum Liefern der Daten an das erste Steuermittel (23, 51) aufweist, um eine Funktion, die sich auf den Betrieb des tragbaren Kommunikationsgeräts bezieht, zu beeinflussen.

2. Gerät nach Anspruch 1, wobei das Signalliefermittel (41, 43) vom Sendeempfänger (7a) getrennt ist.

3. Gerät nach Anspruch 1, wobei das Signalliefermittel (7a) ein Funkfrequenz-Trägersignalliefermittel ist, welches ein Trägersignalgenerator des Sendeempfängers ist.

4. Gerät nach einem der vorhergehenden Ansprüche, wobei das zweite Körperteil (3) abnehmbar ist.

5. Gerät nach einem der vorhergehenden Ansprüche, wobei die Kopplung zwischen dem ersten und dem zweiten Kopplungsmittel (LC1 und LC2) induktiv oder kapazitiv ist.

6. Gerät nach einem der vorhergehenden Ansprüche, wobei das Signalliefermittel (41, 43) ein Signalunterbrechungsmittel zum periodischen Variieren des Signals beinhaltet, und die Datenspeichervorrichtung (17) ein Taktsignalableitungsmittel (600, 600') zum Ableiten eines Taktsignals für die Datenspeichervorrichtung (17) auf Basis der periodischen Variierungen aufweist.

7. Gerät nach einem der Ansprüche 1 bis 5, wobei die Datenspeichervorrichtung (17) ein Taktsignalerzeugungsmittel zum Erzeugen eines Taktsignals für die Datenspeichervorrichtung (17) in Antwort auf das Ableiten einer Stromzufuhr durch das Stromzufuhrsableitungsmittel aufweist.

8. Gerät nach Anspruch 7, wobei das Taktsignalerzeugungsmittel einen fehlenden Impulsdetektor aufweist.

9. Gerät nach einem der vorhergehenden Ansprüche, wobei der Speicher (59) ein beschreibbarer Speicher (59) ist, das Signalliefermittel (41, 43) ein Datenliefermittel (400) zum Modulieren des Trägersignals gemäß der in den Speicher (59) zu schreibenden Daten beinhaltet und die Datenspeichervorrichtung (17) Schreibmittel (360, 330) zum Ableiten von Daten aus dem modulierten Trägersignal und zum Schreiben der abgeleiteten Daten in den Speicher (59) aufweist.

10. Gerät nach Anspruch 9, wobei das erste Steuermittel (23, 51) Nutzungsinformation-Ableitungsmittel aufweist, um Information betreffend die Nutzung des tragbaren Kommunikationsgeräts zu erhalten, und dazu dient, das zu modulierende Trägersignal gemäß den Nutzungsdaten zu modulieren, um den Schreibmitteln (360, 330) zu ermöglichen, die Nutzungsdaten in den Speicher (59) zu schreiben.

11. Gerät nach einem der vorhergehenden Ansprüche, wobei das erste Steuermittel (23, 51) dazu dient, das Signalliefermittel (41, 43) dazu zu veranlassen, das Trägersignal zu einer vorherbestimmten Zeit zu liefern, wie zum Beispiel nach dem Hochfahren des Geräts, wenn das zweite Körperteil (3) erstmalig an das erste Körperteil (5) gekoppelt wird oder in vorherbestimmten Intervallen.

12. Gerät nach einem der vorhergehenden Ansprüche, wobei die im Speicher (59) gespeicherten Daten mindestens folgende Daten beinhalten: Softwaredaten, umfassend mindestens entweder Betriebs-Softwaredaten für das tragbare Kommunikationsgerät oder Game-Softwaredaten, Identitätsdaten, Zugriffsdaten, um dem ersten Steuermittel (23, 51) den Zugriff auf Betriebs-Softwaredaten oder Game-Softwaredaten zu gestatten, Identitätsdaten und Informationsdaten, umfassend mindestens entweder Grafikdaten, Audiodaten, Bilddaten, Videodaten oder Textdaten.

13. Gerät nach einem der vorhergehenden Ansprüche, wobei das Benutzerbetriebsmittel (25) mit dem zweiten Körperteil (3) verknüpft sind.

14. Gerät nach einem der Ansprüche 1 bis 8, wobei das Benutzerbetriebsmittel (25) vom zweiten Körperteil (3) getragen wird und die Datenspeichervorrichtung (17) dazu dient, in Antwort auf eine Benutzereingabe über das Benutzerbetriebsmittel (25) Eingabedaten betreffend den Benutzerbetrieb des Benutzerbetriebsmittels (25) an das erste Körperteil (5) liefert.

15. Gerät nach Anspruch 14, wobei die Datenspeichervorrichtung (17) dazu dient, Anweisungen an das erste Steuermittel (23, 51) zu liefern, um eine Anzeige (13) des tragbaren Kommunikationsgeräts zum Einsatz in einer Anzahl verschiedener Orientierungen zu konfigurieren.

16. Gerät nach Anspruch 13, 14 oder 15, wobei das Benutzerbetriebsmittel (25) mindestens eine Tastatur, ein Tastenfeld, ein Touch-Screen, ein Joystick, eine Digitalisierungstabelle oder ein anderes Eingabemittel ist.

17. Gerät nach einem der Ansprüche 13 bis 16, ferner umfassend eine separate Benutzeroberflächenvorrichtung mit einem auf andere Weise konfigurierten Benutzerbetriebsmittel, wobei die Benutzeroberflächenvorrichtung ebenfalls eine passive Datenspeichervorrichtung, ein Stromableitungsmittel zum Ableiten einer Stromzufuhr, wenn Kopplungsmittel der Benutzeroberflächenvorrichtung an das Kopplungsmittel des tragbaren Kommunikationsgeräts gekoppelt werden, und ein Datenliefermittel zur Lieferung von, in Antwort auf die Betätigung der Benutzereingabevorrichtung durch einen Benutzer, Benutzereingabendaten an das erste Steuermittel (23, 51) aufweist, wenn die Benutzeroberflächenvorrichtung an das tragbare Kommunikationsgerät gekoppelt wird.

18. Gerät nach einem der vorhergehenden Ansprüche, wobei die im Speicher (59) gespeicherten Daten Daten sind, die dem ersten Steuermittel (23, 51) ermöglichen, das zweite Körperteil (3) zu identifizieren, und das erste Steuermittel (23, 51) dazu dient, mindestens eine Funktion oder eine Betriebseigenschaft des tragbaren Kommunikationsgeräts zu deaktivieren, wenn das erste Steuermittel (23, 51) keine Identifikationsdaten oder nicht korrekte Identifikationsdaten empfängt.

19. Gerät nach einem der vorhergehenden Ansprüche, wobei das oder ein zweites Körperteil (3) einen Orientierungssensor trägt, und das zweite Steuermittel dazu dient, in Antwort auf ein Orientierungssignal vom Orientierungssensor, dem ersten Steuermittel (23, 51) Daten, betreffend die Orientierung des Geräts, mitzuteilen, um zu bewirken, dass das erste Steuermittel (23, 51) die Orientierung eines Anzeigebildschirms dementsprechend steuert.

20. Gerät nach einem der vorhergehenden Ansprüche, wobei das zweite Körperteil (3) eine Anzeige des Geräts trägt.

21. Gerät nach einem der vorhergehenden Ansprüche, wobei das oder ein zweites Körperteil (3) anstatt der oder zusätzlich zu der Datenspeichervorrichtung (17) mindestens einen Umgebungssensor trägt, der mindestens auf zum Beispiel Temperatur, Druck, Orientierung, Position, Höhe, Feuchtigkeit, Licht, Ton, eine Chemikalie, eine Flüssigkeit, Wasser, ein Magnetfeld oder Geruch anspricht, und die Datenspeichervorrichtung (17) dazu dient, dem ersten Steuermittel (23, 51) Daten mitzuteilen, die sich auf eine Ausgabe des Sensors beziehen, um den Betrieb des Geräts zu beeinflussen.

22. Gerät nach einem der vorhergehenden Ansprüche, wobei das zweite Körperteil (3) die Fassade des Geräts ist.

23. Gerät nach einem der vorhergehenden Ansprüche, wobei das erste Körperteil (5) das Hauptkörperteil ist, und das zweite Körperteil (3) ein Gehäuseteil des oder ein Zubehör für das Hauptkörperteil ist.

## Revendications

1. Dispositif de communications portable comportant une première partie de corps (5) et une seconde partie de corps (3), dans lequel :
la première partie de corps (5) comporte un émetteur-récepteur pour autoriser une émission et une réception de communications par un réseau de télécommunications mobiles, un premier moyen de commande (23, 51) destiné à commander le fonctionnement du dispositif et un moyen (41, 43 ou 7a) de fourniture de signal destiné à fournir un signal porteur ;
la première partie de corps (5) et la seconde partie de corps (3) comportent des premier et second moyens respectifs (LC1 et LC2) de couplage sans fil agencés à proximité étroite l'un de l'autre pour permettre un couplage sans fil du signal porteur du premier moyen (LC1) de couplage sans fil au second moyen (LC2) de couplage sans fil ;
la seconde partie de corps (3) porte un dispositif (17) de stockage de données ayant une mémoire (59) stockant des données, un moyen (PD) de dérivation d'alimentation électrique destiné à dériver une alimentation électrique du signal porteur couplé au second moyen (LC2) de couplage sans fil, un second moyen de commande (600, 600') destiné à lire des données dans la mémoire (59) en réponse à une dérivation d'une alimentation électrique par le moyen (PD) de dérivation d'alimentation électrique, et un moyen de modulation (M) destiné à moduler le signal porteur en fonction des données lues dans la mémoire (59) ;
la première partie de corps (5) comporte un moyen (45) d'extraction de données destiné à extraire des données à partir de la modulation du signal porteur et un moyen (400) de fourniture de données destiné à fournir les données au premier moyen de commande (23, 51) afin d'affecter une fonction portant sur le fonctionnement du dispositif de communications portable.

2. Dispositif selon la revendication 1, dans lequel le moyen (41, 43) de fourniture de signal est séparé de l'émetteur-récepteur (7a).

3. Dispositif selon la revendication 1, dans lequel le moyen (7a) de fourniture de signal comporte un moyen de fourniture d'un signal porteur à fréquence radio comportant un générateur de signal porteur de l'émetteur-récepteur.

4. Dispositif selon l'une quelconque des revendications précédentes, dans lequel la seconde partie de corps (3) est amovible.

5. Dispositif selon l'une quelconque des revendications précédentes, dans lequel le couplage entre les premier et second moyens de couplage (LC1 et LC2) est inductif ou capacitif.

6. Dispositif selon l'une quelconque des revendications précédentes, dans lequel le moyen (41, 43) de fourniture de signal comprend un moyen d'interruption de signal destiné à faire varier périodiquement le signal, et le dispositif (17) de stockage de données comporte un moyen (600, 600') de dérivation de signal d'horloge destiné à dériver un signal d'horloge pour le dispositif (17) de stockage de données sur la base des variations périodiques.

7. Dispositif selon l'une quelconque des revendications 1 à 5, dans lequel le dispositif (17) de stockage de données comporte un moyen de génération de signal d'horloge destiné à générer un signal d'horloge pour le dispositif (17) de stockage de données en réponse à une dérivation d'une alimentation électrique par le moyen de dérivation d'alimentation électrique.

8. Dispositif selon la revendication 7, dans lequel le moyen de génération de signal d'horloge comporte un détecteur d'impulsion manquante.

9. Dispositif selon l'une quelconque des revendications précédentes, dans lequel la mémoire (59) est une mémoire inscriptible (59), le moyen (41, 43) de fourniture de signal comprend un moyen (400) de fourniture de données destiné à moduler le signal porteur en fonction de données devant être écrites dans la mémoire (59), et le dispositif (17) de stockage de données comporte un moyen d'écriture (360, 330) destiné à dériver les données à partir du signal porteur modulé et à écrire les données dérivées dans la mémoire (59).

10. Dispositif selon la revendication 9, dans lequel le premier moyen de commande (23, 51) comporte un moyen de dérivation d'information d'utilisation destiné à obtenir une information concernant l'utilisation du dispositif de communications portable et peut fonctionner pour amener le signal porteur à être modulé en fonction des données d'utilisation afin de permettre au moyen d'écriture (360, 330) d'écrire les données d'utilisation dans la mémoire (59).

11. Dispositif selon l'une quelconque des revendications précédentes, dans lequel le premier moyen de commande (23, 51) peut être mis en oeuvre pour amener le moyen (41, 43) de fourniture de signal à fournir le signal porteur à un temps prédéterminé tel qu'à la mise sous tension du dispositif, lorsque la seconde partie de corps (3) est accouplée en premier à la première partie de corps (5) ou à intervalles prédéterminés.

12. Dispositif selon l'une quelconque des revendications précédentes, dans lequel les données stockées dans la mémoire (59) comprennent au moins l'une de : données de logiciel comportant au moins l'une de données d'un logiciel d'exploitation pour le dispositif de communications portable et de données de logiciel de jeux, de données d'identité, de données d'accès pour autoriser le premier moyen de commande (23, 51) à accéder à des données de logiciel d'exploitation ou à des données de logiciel de jeux, des données d'identité et des données d'information comprenant au moins l'une de données graphiques, de données audio, de données d'image, de données vidéo et de données de texte.

13. Dispositif selon l'une quelconque des revendications précédentes, dans lequel un moyen d'exploitation par l'utilisateur (25) est associé à la seconde partie de corps (3).

14. Dispositif selon l'une quelconque des revendications 1 à 8, dans lequel un moyen d'exploitation par l'utilisateur (25) est porté par la seconde partie de corps (3) et le dispositif (17) de stockage de données peut être mis en oeuvre, en réponse à une entrée de l'utilisateur par l'intermédiaire du moyen (25) d'exploitation par l'utilisateur, pour fournir des données d'entrée concernant l'exploitation par l'utilisateur du moyen (25) d'exploitation par l'utilisateur à la première partie de corps (5).

15. Dispositif selon la revendication 14, dans lequel le dispositif (17) de stockage de données peut être mis en oeuvre pour fournir des instructions au premier moyen de commande (23, 51) afin de configurer un afficheur (13) du dispositif de communications portable pour une utilisation dans l'une d'un certain nombre d'orientations différentes.

16. Dispositif selon la revendication 13, 14 ou 15, dans lequel le moyen (25) d'exploitation par l'utilisateur comporte au moins l'un d'un clavier, d'un pavé de touches, d'un écran tactile, d'une manette de commande, d'une table de numérisation et d'une autre entrée.

17. Dispositif selon l'une quelconque des revendications 13 à 16, comportant en outre une interface d'utilisateur séparée ayant une forme différente de configuration du moyen d'exploitation par l'utilisateur, le dispositif d'interface d'utilisateur ayant aussi un dispositif de stockage passif de données, un moyen de dérivation d'énergie destiné à dériver une alimentation électrique lorsque des moyens d'accouplement du dispositif d'interface d'utilisateur sont accouplés au moyen d'accouplement du dispositif de communications portable et un moyen de fourniture de données destiné à fournir, en réponse à une opération effectuée par un utilisateur du dispositif d'entrée d'utilisateur, des données d'entrée d'utilisateur au premier moyen de commande (23, 51) lorsque le dispositif d'interface d'utilisateur est couplé au dispositif de communications portable.

18. Dispositif selon l'une quelconque des revendications précédentes, dans lequel les données stockées dans la mémoire (59) comprennent des données autorisant le premier moyen de commande (23, 51) à identifier la seconde partie de corps (3) et le premier moyen de commande (23, 51) peut être mis en oeuvre pour empêcher au moins une fonction ou une caractéristique de fonctionnement du dispositif de communications portable si le premier moyen de commande (23, 51) ne reçoit pas de données d'identification ou ne reçoit pas de données d'identification correctes.

19. Dispositif selon l'une quelconque des revendications précédentes, dans lequel la ou une seconde partie de corps (3) porte un capteur d'orientation et le second moyen de commande peut être mis en oeuvre, en réponse à un signal d'orientation provenant du capteur d'orientation, pour communiquer au premier moyen de commande (23, 51) des données concernant l'orientation du dispositif afin d'amener le premier moyen de commande (23, 51) à commander en conséquence l'orientation d'un écran d'affichage.

20. Dispositif selon l'une quelconque des revendications précédentes, dans lequel la seconde partie de corps (3) porte un afficheur du dispositif.

21. Dispositif selon l'une quelconque des revendications précédentes, dans lequel la ou une seconde partie de corps (3) porte, au lieu ou en plus du dispositif (17) de stockage de données, au moins un capteur d'environnement qui est sensible, par exemple, à au moins l'un d'une température, d'une pression, d'une orientation, d'une position, d'une hauteur, de l'humidité, de la lumière, d'un son, d'une substance chimique, d'un fluide, de l'eau, d'un champ magnétique, d'une odeur et le dispositif (17) de stockage de données peut être mis en oeuvre pour communiquer au premier moyen de commande (23, 51) des données concernant un signal de sortie du capteur afin d'affecter le fonctionnement du dispositif.

22. Dispositif selon l'une quelconque des revendications précédentes, dans lequel la seconde partie de corps (3) comporte une façade du dispositif.

23. Dispositif selon l'une quelconque des revendications précédentes, dans lequel la première partie de corps (5) comprend une partie de corps principale et la seconde partie de corps (3) comprend une partie de boîtier de, ou un accessoire pour, la partie de corps principale.
